# FASCICULE DE BREVET EUROPEEN

(11) **EP 1 107 771 B1**
(45) Date de publication et mention de la délivrance du brevet: **12.06.2002**
(21) Numéro de dépôt: 99941680.3
(22) Date de dépôt: 26.08.1999
(51) Int. Cl.: A61K 35/56

(54) **MEDICAMENT HOMEOPATHIQUE COMPRENANT UN OU PLUSIEURS COMPOSANTS DE LA NACRE DONT LA BIO-ARAGONITE**
EINE ODER MEHRERE KOMPONENTE DES PERLMUTTES EINSCHLIESSLICH BIO-ARAGONIT ENTHALTENDES HOMEOPATHISCHES ARZNEIMITTEL
HOMEOPATHIC MEDICINE COMPRISING ONE OR SEVERAL MOTHER-OF-PEARL CONSTITUENTS INCLUDING BIO-ARAGONITE

(30) Priorité: 27.08.1998 FR 9810857
(43) Date de publication de la demande: 20.06.2001
(73) Titulaire: Regent Genus Organisms International Ltd., London WIY 9PA (GB)
(72) Inventeur: LABLANCHY, Jean-Pierre, F-75017 Paris (FR)
(74) Mandataire: Bonneau, Gérard
(86) Numéro de dépôt international: FR9902048
(87) Numéro de publication internationale: WO0012104

(56) Documents cités:
- EP-A- 0 393 267
- WO-A-97/23231
- BOERICKE W.: 'Handbuch der Homöopathischen Materia Medica, pp. 178-179', 1996, KARL F. HAUG VERLAG, HEIDELBERG
- VOISIN H.: 'Materia Medica des Homöopathischen Praktikers', 1991, KARL F. HAUG VERLAG, HEIDELBERG voir page 466

## Description

### Domaine technique

La présente invention concerne un médicament homéopathique comprenant un ou plusieurs composants de la nacre, dont la bio-aragonite. Elle concerne également un procédé de réalisation de ce médicament.

### Etat de la technique

Au sens de la présente invention, on entend par "médicament homéopathique", un médicament administré à faibles doses, comparées à celles administrées en allopathie, c'est à dire à une dilution minimale de la teinture mère de 1DH (un dixième).

La nacre, ou aragonite chonchylifère, est une formation minéralisée biogène, constituée essentiellement d'une partie minérale consistant en du carbonate de calcium cristallisé sous forme d'aragonite et essentiellement d'une matrice organique de substances fibreuses et non fibreuses, représentant environ 1 à 2% de la masse totale. La nacre peut être obtenue à partir de coquilles de mollusques nacriers, notamment de certaines huîtres telles que *Pinctada maxima,* dont elle constitue la couche la plus interne.

Le document FR 2 742 661 divulgue une utilisation de compositions contenant de la nacre, en vue de la réalisation d'une préparation pharmaceutique ou cosmétologique destinée à assurer la synthèse de nouvelles cytokératines.

On connaît par ailleurs les propriétés de la coquille d'huître dans la supplémentation calcique et la très bonne absorption intestinale d'électrolysats de coquilles d'huîtres comparée à celle du carbonate de calcium. (Takuo Fujita et all, Intestinal absorption of oyster shell electrolysate, Bone and Mineral, 4, p. 321-327 (1988).

Plus précisément, il a été démontré que l'absorption orale de coquilles d'huîtres chauffées en vacuum ("in vacuo") permettait d'augmenter chez l'homme la densité minérale osseuse de la colonne vertébrale de façon beaucoup plus importante qu'une préparation à base de carbonate de calcium (Takuo Fujita et all., Heated Oyster Shell - Seaweed Calcium (AAA Ca) on Osteoporesis, Calcif. Issue Int. 58:226-230 (1996).

Le document FR 2 743 075 divulgue quant à lui un procédé de préparation de substances biologiquement actives à partir de la nacre, ce procédé étant essentiellement caractérisé par le fait que l'on extrait une fraction hydrosoluble à partir de poudre de nacre.

Enfin, on connaît les spécialités homéopathiques CALCAREA OSTREICA et CALCAREA CARBONICA OSTREARUM, toutes deux à base de calcaire d'huître.

Ainsi, la coquille d'huître et certains extraits de nacre ont déjà été proposés pour des utilisations thérapeutiques, mais cet art antérieur concerne des compositions pharmaceutiques utilisées à dose allopathique.

### Exposé de l'invention

Il n'a jamais été envisagé d'utiliser la bio-aragonite de nacre issue de mollusques tels que l'huître *Pinctada Maxima,* dans laquelle le carbonate de calcium se présente sous forme orthorhombique.

La société demanderesse a constaté que cette bio-aragonite de nacre offre des avantages importants en thérapeutique, en particulier dans le traitement de la douleur et plus particulièrement encore dans le traitement des douleurs causées par des pathologies ou des traumatismes osseux.

La présente invention a donc pour objet un médicament homéopathique caractérisé par le fait qu'il comprend au moins un composant de la nacre, la bio-aragonite de nacre biocompatible, à une dilution minimale de 1 DH. Ce médicament est préférentiellement utilisé pour le traitement des douleurs osseuses et notamment des douleurs liées aux maladies osseuses dues à une pathologie, aux carences oestrogéniques de la pré et post ménopause, au vieillissement, au cancer ou à un traumatisme - aux affections cartilagineuses notamment l'arthrose.

### Description détaillée de l'invention

On utilise donc la bio-aragonite de nacre, c'est à dire la nacre comprenant à la fois sa partie minérale et organique, de préférence sous forme micronisée. La bio-aragonite est rendue biocompatible par inhibition de la partie la plus immunogène de la substance organique de la bio-aragonite, afin de ne maintenir bioactive que la partie de la substance organique en association avec le minéral susceptible d'avoir un effet positif.

Des essais ont été réalisés sur des patients présentant des douleurs arthrosiques (arthroses rachidienne, diffuse, post-traumatique). Sur une période de 60 jours, on a administré à ces patients par voie nasale un médicament homéopathique à base de bio-aragonite de nacre biocompatible, dilué à 3DH, c'est à dire trois dilutions successives au dixième, à raison de une dose deux fois par semaine.

On a ensuite mesuré sur une échelle de la douleur graduée de 0 (pas de douleur) à 10 (maximum de douleur), l'évolution de la douleur entre les jours J0, J30 et J60.

Ces résultats sont présentés sur le tableau 1 ci-après, qui présente l'évaluation de la douleur par 13 patients aux jours J0, J30 et J60 et le facteur de division de cette douleur entre J0 et J60 :

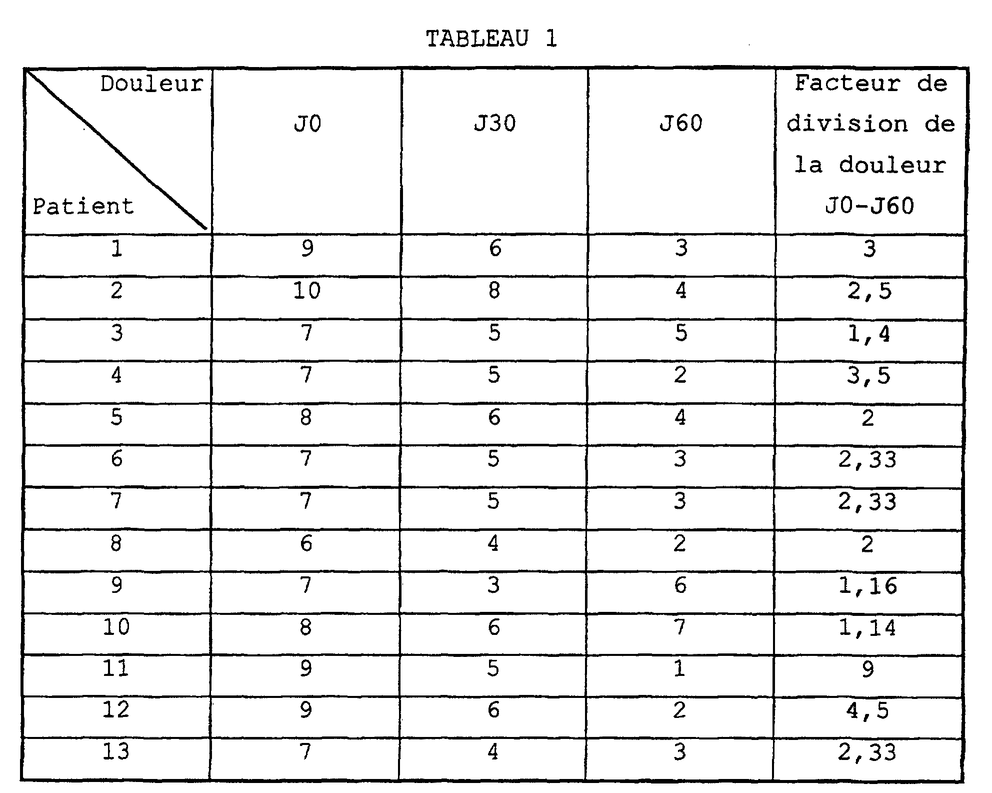

Ces résultats montrent donc un facteur moyen de division de la douleur entre J0 et J60 de 2,86.

Seuls 3 individus sur 13 présentent un facteur de division de la douleur entre J0 et J60 inférieur à 2.

Ces résultats mettent donc bien en évidence l'efficacité de telles compositions homéopathiques.

La présente invention a également pour objet un procédé de préparation de médicament homéopathique caractérisé par le fait que l'on procède à une dilution et/ou à une suspension du ou des composants choisis de la nacre avec un excipient pharmaceutiquement acceptable, qui peut consister notamment en un excipient ou en un solvant, et qu'on met en forme le mélange dilué ainsi obtenu.
Cette dilution est réalisée suivant les techniques conventionnelles et les normes de qualité mises en oeuvre dans l'industrie homéopathique. Les médicaments homéopathiques sont ensuite présentés sous une forme galénique et conditionnés en fonction du mode d'administration désiré, qui peut être oral, perlingual, nasal ou sous forme injectable.

Le plus généralement, les médicaments ont un niveau de dilution de 1DH à 100K (KORSAKOFF).

De préférence, ce niveau de dilution se situe entre 1DH et 30CH (teinture mère diluée au centième successivement trente fois), et plus préférentiellement encore entre 1DH et 1CH.

Les exemples qui suivent illustrent l'invention, sans pour autant la limiter.

### Exemple 1. :

L'excipient utilisé est le saccharose et la bio-aragonite biocompatible présente une dimension granulométrique de 50 à 150 microns.

On dilue 1 gramme de bio-aragonite pour 100 grammes de saccharose, on granule par granulation humide, puis on tamise et on obtient ainsi des granules pour l'administration perlinguale.

### Exemple 2. :

Sous hotte à flux laminaire, on pèse 1 gramme de bio-aragonite biocompatible que l'on mélange à 1 litre de sérum physiologique stérile contenu dans un récipient ouvert au dernier moment.

On referme ensuite le récipient et on remue 20 fois l'ensemble.

Toujours sous hotte à flux laminaire, on prélève à l'aide d'une seringue stérile 10 ml de cette solution mère, que l'on réinjecte à travers la capsule caoutchouc dans un flacon stérile d'un litre de sérum physiologique.

Dans ce même flacon, à l'aide de la même aiguille restée en place dans le caoutchouc et d'une autre seringue, on ajoute 1 ml de benzododécinium pour 1 litre de préparation.

On obtient ainsi une solution en 2DH. Cette solution est ensuite introduite dans un flacon-spray convenant à l'administration pernasale.

## Revendications

1. Médicament homéopathique **caractérisé en ce qu'**il comprend la bio-aragonite de nacre, rendue biocompatible par inhibition de la partie la plus immunogène de la substance organique, à une dilution minimale de 1DH.

2. Médicament homéopathique selon la revendication 1, se présentant sous une forme galénique pour l'administration par voie orale.

3. Médicament homéopathique selon l'une quelconque des revendications 1 ou 2, se présentant sous une forme galénique pour l'administration par vois perlinguale.

4. Médicament homéopathique selon la revendication 1, se présentant sous une forme galénique pour l'administration par voie parentérale.

5. Médicament homéopathique selon la revendication 1 , se présentant sous une forme galénique pour l'administration par voie nasale, notamment sous forme de spray,

6. Procédé de préparation du médicament homéopathique selon l'une quelconque des revendications 1 à 5, **caractérisé en ce qu'**il consiste à diluer et/ou mettre en suspension la bioaragonite de nacre biocompatible avec un excipient pharmaceutiquement acceptable qui peut consister notamment en un diluant ou en un solvant, et à mettre en forme le mélange dilué ainsi obtenu.

7. Procédé de préparation d'un médicament homéopathique selon la revendication 6, **caractérisé en ce que** la bioaragonite biocompatible est utilisée sous forme pulvérulente, à une granulométrie comprise entre 50 et 150 microns.

8. Utilisation de la bioaragonite de nacre biocompatible, pour la préparation d'un médicament homéopathique utilisé pour le traitement de la douleur.

9. Utilisation de la bioaragonite de nacre biocompatible, pour la préparation d'un médicament homéopathique utilisé pour le traitement de la douleur d'origine osseuse et notamment liée aux maladies osseuses dues à une pathologie, aux carences oestrogéniques de la pré et post ménopause, au vieillissement, au cancer ou à un traumatisme - aux affections cartilagineuses notamment l'arthrose.

## Claims

1. A homeopathic medication **characterized in that** it includes mother-of-pearl bio-aragonite which is rendered biocompatible through the inhibition of the most immunogenic part of the organic substance, at a minimal dilution of 1DH.

2. The homeopathic medication according to claim 1, presented in a galenical form for oral administration.

3. The homeopathic medication according to any one of claims 1 or 2, presented in a galenical form for perlingual administration.

4. The homeopathic medication according to claim 1, presented in a galenical form for parenteral administration.

5. The homeopathic medication according to claim 1, presented in a galenical form for nasal administration, particularly in the form of a spray.

6. A process for preparing the homeopathic medication according to any one of claims 1 to 5, **characterized in that** it consists in diluting and/or placing in suspension the biocompatible mother-of-pearl bio-aragonite using a pharmaceutical acceptable excipient which can namely consist of a diluting agent or a solvent, and to formulate the diluted mixture thus obtained.

7. The process for preparing the homeopathic medication according to claim 6, **characterized in that** the biocompatible bio-aragonite is used in pulverulent form, having a particle size between 50 and 150 microns.

8. The use of the biocompatible bio-aragonite for the preparation of a homeopathic medication for treating pain.

9. The use of the biocompatible mother-of-pearl bio-aragonite for the preparation of a homeopathic medication used for treating pain of bone origin and in particular related to bone diseases caused by a pathology, pre-menopause or post-menopause estrogenic deficiency, ageing, cancer or trauma and to cartilaginous disorders particularly osteoarthritis.

## Patentansprüche

1. Homöopathisches Arzneimittel, **dadurch gekennzeichnet, dass** es Bio-Aragonit von Perlmutter, das durch Hemmung des am stärksten immunogenen Teils der organischen Substanz biokompatibel gemacht wurde, in einer Mindestverdünnung von 1 Zehntel enthält.

2. Homöopathisches Arzneimittel nach Anspruch 1 in einer galenischen Form für die orale Verabreichung.

3. Homöopathisches Arzneimittel nach einem der Ansprüche 1 oder 2 in einer galenischen Form für die perlinguale Verabreichung.

4. Homöopathisches Arzneimittel nach Anspruch 1 in einer galenischen Form für die parenterale Verabreichung.

5. Homöopathisches Arzneimittel nach Anspruch 1 in einer galenischen Form für die nasale Verabreichung, insbesondere in Form von Spray.

6. Verfahren zur Herstellung eines homöopathischen Arzneimittels nach einem der Ansprüche 1 bis 5, **dadurch gekennzeichnet, dass** es darin besteht, dass man das biokompatible Bio-Aragonit von Perlmutter mit einem pharmazeutisch verträglichen Träger verdünnt und/oder in Suspension bringt, der insbesondere aus einem Verdünnungsmittel oder einem Lösungsmittel bestehen kann, und die auf diese Weise erhaltene verdünnte Mischung in eine Form bringt.

7. Verfahren zur Herstellung eines homöopathischen Arzneimittels nach Anspruch 6, **dadurch gekennzeichnet, dass** biokompatibler Bio-Aragonit in Pulverform mit einer Korngröße zwischen 50 und 150 Mikron verwendet wird.

8. Verwendung von biokompatiblem Bio-Aragonit von Perlmutter für die Herstellung eines homöopathischen Arzneimittels für die Schmerztherapie.

9. Verwendung von biokompatiblem Bio-Aragonit von Perlmutter für die Herstellung eines homöopathischen Arzneimittels für die Behandlung von Knochenschmerzen, die insbesondere mit Knochenerkrankungen infolge von pathologischen Veränderungen, infolge von Östrogenmangel der Prä- und Postmenopause, Altern, Krebs oder Verletzungen - infolge von Knorpelerkrankungen, insbesondere Arthrose, verbunden sind.
